# EUROPEAN PATENT SPECIFICATION

(11) **EP 3 681 378 B1**
(45) Date of publication and mention of the grant of the patent: **23.07.2025**
(21) Application number: 18769465.8
(22) Date of filing: 11.09.2018
(51) Int. Cl.: A61B 5/00

(54) **FINGER RING FOR SENSING PHYSIOLOGICAL AND/OR BEHAVIOURAL PARAMETERS OF A USER**
FINGERRING ZUR ERFASSUNG VON PHYSIOLOGISCHEN UND/ODER VERHALTENSPARAMETERN EINES BENUTZERS
BAGUE POUR DÉTECTER DES PARAMÈTRES PHYSIOLOGIQUES ET/OU DE COMPORTEMENT D'UN UTILISATEUR

(30) Priority: 12.09.2017 GB 201714653; 29.03.2018 GB 201805188
(43) Date of publication of application: 22.07.2020
(73) Proprietor: Braintrain2020 Limited, Sheffield, South Yorkshire S3 7HQ (GB)
(72) Inventor: MILLS, Richard, Sheffield South Yorkshire S6 1RU (GB); VAN DE WERKEN, Maan, Sheffield South Yorkshire S10 4NF (GB); IRONMONGER, Paul, Doncaster South Yorkshire DN12 3QL (GB); HALL, Richard, Harrogate Yorkshire HG3 5SN (GB); FLOOD, Tim, Stockton-On-Tees Durham TS18 5LB (GB)
(74) Representative: HGF
(86) International application number: PCT/GB2018/052571
(87) International publication number: WO 2019/053411

(56) References cited:
- WO-A1-01/03574
- WO-A1-2011/053235
- US-A1- 2010 168 531
- US-A1- 2013 226 015
- US-B1- 6 672 105
- US-B1- 7 797 964
- US-B1- 9 711 060

## Description

### TECHNICAL FIELD

The present invention relates to a ring for sensing one or more physiological and/or behavioural parameters of a user. Further aspects of the invention relate to a kit.

### BACKGROUND

In the Information Age, many applications or scenarios now require obtaining information relating to one or more physiological and/or behavioural parameters of a subject, such as a human. Such information may be obtained, for example, by measuring or sensing one or more signals indicative of said one or more parameters.

When measuring or sensing signals indicative of some parameters, such as heart rate for example, contact between a subject's skin and a sensing means, such as a sensor, may be required. In order to ensure a reasonable level of accuracy, an optimal amount of contact must be provided between the sensor and the subject. For example, when measuring heart rate by means of an optical sensor, there must be sufficient contact between the subject's skin and the sensor to prevent excessive ambient light from reaching the sensor and affecting the measurement. However, too forceful a contact between the sensor and the subject may result in compression of adjacent blood vessels of the subject, affecting fluid flow therein, which may result in an inaccurate measurement.

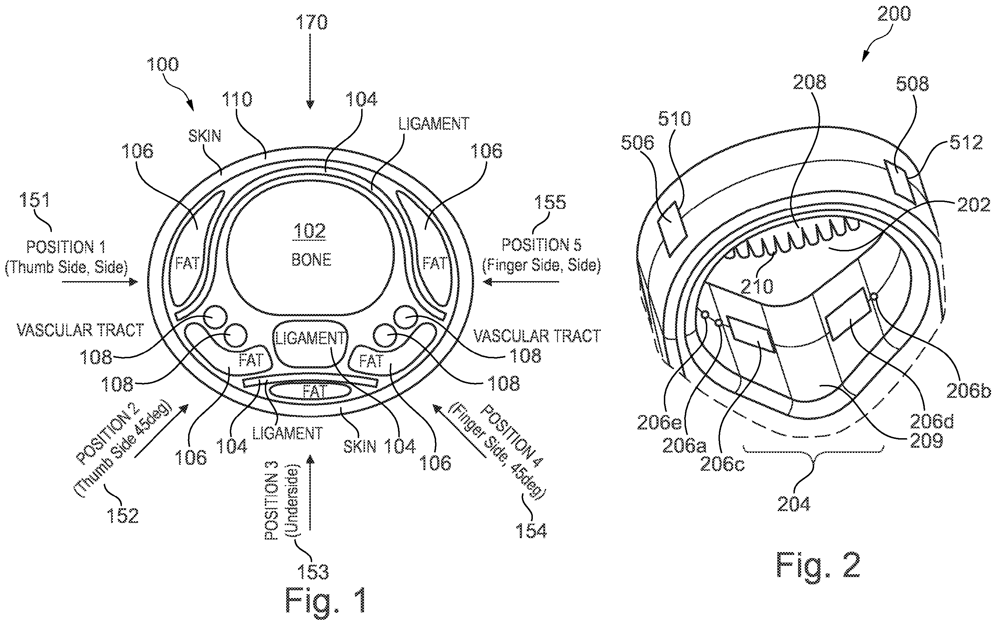

Measuring or sensing signals of the subject via parts of the anatomy conventionally, such as the subject's chest or wrist, may be intrusive or at least uncomfortable for the subject. However, information obtained via less intrusive parts of the subject's anatomy, such as a finger, may suffer from reduced accuracy, due to anatomical complexity and/or anatomical obstructions hindering the measuring or sensing. Figure 1 shows a schematic section through a human finger 100. The finger 100 comprises a bone 102 and vascular tracts 108, encapsulated within a sheath of skin 110. A number of ligaments 104 and fat pockets 106 obstruct the vascular tracts 108. The vascular tracts 108 may easily be compressed, particularly due to the presence of the bone 102, which has a much higher Young's modulus, relative to soft connective tissues such as the surrounding fat 106, ligaments 104, vascular tracts 108 and skin 110.

Sleep disorders, including difficulties in either falling asleep or remaining asleep, are increasingly common; one in three adults in the UK are reported to suffer from a sleep disorder of some kind. A lack of sleep can result in impaired concentration and lengthened reaction times whilst awake, as well as a general feeling of tiredness. Difficulties in falling asleep can be caused by an inability to ignore conscious thoughts, which may be caused by stress or anxiety.

Some approaches for inducing sleep include meditative approaches in which a stimulus is provided to encourage the subject to relax, for example by providing a light whose intensity varies cyclically so that the subject may match the frequency of their breathing to frequency of the cycles of the light. In this way the frequency of the cycles of the light may be gradually reduced so that the subject is encouraged to slow their breathing at a predetermined rate. An example of such an approach is that implemented by the Nightwave^{™} sleep assistant (http://www.nightwave.co.uk/index.htm ).

Such approaches have the advantage that they have very limited side effects. However, they have the disadvantage that they do not engage with or adapt to the subject, so they may be ineffective for certain individuals or on occasions when the subject finds it particularly difficult to get to sleep.

Another approach to improving sleep involves taking physiological measurements from the subject and attempting to manipulate their surrounding environment to improve sleep. Although such approaches may help the subject to sleep better once they have fallen asleep, they are of limited usefulness in inducing the subject to fall asleep in the first instance.

WO2015/040373 relates to an apparatus for inducing sleep capable of sequentially providing a subject with a stimulus, each stimulus based on an active input provided via an input means by the subject in response to a previous stimulus.

US9711060B1 relates to a biometric sensing apparatus for estimating the emotional state of a user. A self-contained biometric sensing ring is worn on the finger (or fingers) of the user during their normal activities. Biometric information is collected and sent wirelessly to the user's mobile device configured with application programming for analyzing and displaying the biometric information. The biometric ring is configured with sensors that the structure of the ring retains at a proper pressure against the finger of the user.

WO2011053235A1 relates to an electronic finger ring for wireless, 4-dimensional remote steering and/or monitoring.

US6672105B1 relates to a finger ring fit adjuster permits a finger ring to be slid onto a finger and over a knuckle and then adjust to a snug fit.

Embodiments of the present disclosure aim to provide an improved input means for prior art sleep inducing apparatuses. Embodiments of the present disclosure aim to provide apparatus and components thereof for inducing sleep, which apparatus at least partially mitigates the problems described above.

### SUMMARY OF THE INVENTION

The present invention is defined by the appended claims.

According to an aspect of the invention, there is provided a ring for sensing one or more physiological and/or behavioural parameters of a user, the ring comprising:
an aperture for receiving a finger of the user therethrough, the aperture comprising:
a sensing portion comprising a substantially V-shaped portion;
one or more sensing means disposed in the sensing portion; and
a pad opposing the sensing portion,
wherein the pad is arranged to contact a finger to urge the finger against the one or more sensing means when the finger is received in the aperture.

Advantageously, the ring enables a finger of a user to contact the sensing means with an optimal force, without obstructing or compressing anatomical structures within the finger. This improves the accuracy and reliability of sensed physiological and/or behavioural parameters. Advantageously, a number of physiological parameters are best measured of monitored at a finger of a user. For example, heart rate may be best measured at a fingertip. Skin temperature and galvanic skin response may also be best measured or monitored at a finger of a user. Fingers are also advantageously low in friction.

In some embodiments, behavioural parameters or characteristics may comprise voluntary behavioural parameters, such as steps taken throughout the day, and/or involuntary behavioural parameters, such as a duration of light exposure or sleep characteristics, such as movement during sleep.

According to another aspect of the disclosure, there is provided a ring for sensing one or more physiological and/or behavioural parameters of a user, the ring comprising:
an aperture for receiving a finger of the user therethrough, the aperture comprising:
a sensing portion;
one or more sensing means disposed in the sensing portion; and
a resiliently deformable pad opposing the sensing portion,
wherein the resiliently deformable pad is arranged to contact a finger to urge the finger against the one or more sensing means when the finger is received in the aperture.

In some embodiments the sensing portion comprises a substantially V-shaped portion. Surprisingly, the V-shaped portion increases a finger surface area contacting the sensing portion of the ring when the finger is received in the aperture, compared to prior art ring arrangements is less likely when placing a random finger size on an internal circumference of a prior art ring arrangement having a circular aperture, which may result in small gaps at either side of the finger, for example, due to a mismatch between a diameter of the circular aperture and a diameter of the finger. This advantageously increases an area within which to provide said sensing means. The V-shaped portion may comprise an apex opposed to a central portion of the pad.

In some embodiments, the pad is removable. Advantageously, this enables the replacement of the pad, for example with a pad of different dimensions. This enables the ring to be used by a variety of users having any variety of finger shapes or sizes. The ability to replace the pad also enables the pad to be removed and cleaned, or replaced with a clean pad, thereby improving hygiene of the ring.

Optionally, the pad is receivable in the ring by means of cooperating male pips and female detents in the pad and the ring. Advantageously, this allows for a secure engagement of the pad in the ring, whilst enabling quick and easy release of the pad when it is desired to replace the pad.

In some embodiments, the pad is resiliently deformable. The resiliently deformable pad may comprise a silicone elastomer. Advantageously, silicone elastomers are readily available. Alternatively, the pad may be non-resilient or substantially rigid.

In some embodiments, the pad comprises a plurality of projections for contacting the finger. Advantageously, the projections increase the surface area of the pad adjacent a finger received in the aperture, which increases a force applied to the finger, thereby helping to hold the finger in place, such as in contact with the sensing means, thereby allowing for uninterrupted sensing of the one or more physiological and/or biological parameters.

In some embodiments, a contact surface of the pad comprises a convex surface or a concave surface. In some embodiments, the pad comprises a through hole. In some embodiments, the pad comprises a bladder of fluid, such as air or a liquid. Advantageously, varying the concavity or convexity of the pad helps to urge the finger received in the aperture of the ring against the sensing portion, or, where the sensing portion comprises a substantially V-shaped portion, the apex of the substantially V-shaped portion, thereby helping to hold the finger in place, such as in contact with the sensing means, thereby allowing for uninterrupted sensing of the one or more physiological and/or biological parameters without obstructing or compressing anatomical structures within the finger.

In some embodiments, the apex of the substantially V-shaped portion comprises an angle of between 85° and 120°. Advantageously, an angle in this range helps to increase a finger surface area contacting the sensing portion of the ring when the finger is received in the aperture. This advantageously increases an area within which to provide said sensing means.

In some embodiments, the apex of the substantially V-shaped portion comprises an angle of approximately 105°. Advantageously, an angle of 105° helps to increase a finger surface area contacting the sensing portion of the ring when the finger is received in the aperture. This advantageously increases an area within which to provide said sensing means.

In some embodiments, the ring comprises active input means for engaging by the user. Advantageously, this enables the user to consciously interact with the ring. Said active input means may comprise a button for pressing by the user. In some embodiments, the ring may comprise means for outputting a stimulus to the user. Advantageously, this enables the device to provide feedback to the user in response to said one or more physiological and/or behavioural parameters of the user and/or in response to a user interaction with the active input means. It will be appreciated that, in some embodiments, said sensing means itself may function as the active input means. For example, a user could make a conscious decision to take a deep breath in and/or a deep breath out, which may be sensed by said sensing means in the sensing portion.

In some embodiments, the one or more sensing means comprises any one or more of or any combination of any one or more of: a temperature sensor, a galvanic skin response "GSR" sensor, an optical sensor, a light sensor, an accelerometer, a gyroscope, a force sensor, a grip force sensor, a body temperature sensor, a skin temperature sensor, a sensor for sensing blood oxygen saturation levels (SpO₂), a sensor for sensing mixed venous oxygen saturation (SvO₂), a sensor for sensing a user's reaction time or times, a respiratory rate sensor, a blood pressure sensor, one or more microelectromechanical systems (MEMS) for measuring movement, muscle contraction and/or relaxation, one or more particle sensors, such as a VOx sensor, for sensing a composition or at least one or more constituents, such as alcohol and/or drugs and/or medication and/or metabolites thereof in air exhaled by a user, a sensor arranged to sense electrical brain activity, a microphone and a camera.

Advantageously, such sensing means, amongst others which may be used within the scope of this invention, enable sensing of a variety of useful physiological and/or behavioural parameters of a user. In some embodiments, the sensing means may be arranged to detect and differentiate between both natural and artificial light exposure, and/or to detect and differentiate between ultraviolet (UV) and light emitted by an LED light source, and/or to detect and differentiate different wavelengths of light.

In some embodiments, at least one of the one or more sensing means may extend beyond a threshold of the aperture. Advantageously, this increases a contact surface area between the sensing means and a finger, such as an index finger, of the user. The term "threshold" as used herein may refer to a location adjacent to an internal circumference of the aperture of the ring.

In some embodiments, the ring comprises one or more sensors for measuring one or more attributes of an environment proximal to the ring. Said sensors may optionally comprise any one or more of a temperature sensor for measuring ambient temperature or changes in ambient temperature, a light sensor for sensing ambient light or changes in ambient light levels and a sound sensor or recorder, such as a microphone, for sensing ambient noise or changes in ambient noise levels. Optionally the one or more attributes of an environment may comprise any one or more of ambient temperature, changes in ambient temperature, ambient light levels, changes in ambient light levels and ambient noise levels or changes in ambient noise levels.

In some embodiments, the ring comprises a memory means for storing sensed information. Advantageously, this enables the ring to store information related to sensed said one or more physiological and/or behavioural parameters throughout a period of time, such as a day, a week or a month, for example, for analysis at a later point in time.

In some embodiments, the ring comprises a transmitting means for transmitting sensed information to a remote receiving device. Advantageously, this enables information relating to said one or more physiological and/or behavioural parameters to be collected by the ring and transmitted to another device for analysis or data storage. This lightens demand on any processing means, such as a processor, which may be provided in the ring. The transmitting means also advantageously enables a more compact ring to be provided, improving portability and user comfort when wearing the ring.

According to another aspect of the disclosure, there is provided a trigger device comprising:
a handle for gripping by a user, the handle comprising a proximal end and a distal end; and
the ring of any one or combination of the embodiments described above, the ring extending radially from the handle.

Advantageously, the handle enables a user to grip the device whilst a finger of the user is inserted in the aperture of the ring. This provides a user with more stability and comfort during finger engagement with the ring.

Optionally, the ring extends radially from the proximal end of the handle. Advantageously, this helps to guide a user to hold the device in a correct orientation. This helps to ensure that the user places their finger in the ring in a correct orientation.

In some embodiments, the trigger device comprises an input button disposed at the proximal end of the handle, the button comprising a force sensor. This may be in addition to or alternative to the active input means which may be provided in the ring. Advantageously, this provides an active input means for the user. In some embodiments, the button is arranged to be pressed by a thumb of the user whilst the handle is gripped by fingers of the user.

In some embodiments, the handle comprises one or more sensors for measuring a user's grip strength. Advantageous, this increases the amount of obtainable information relating to said one or more physiological and/or behavioural parameters of a user.

In some embodiments, the trigger device comprises one or more sensors for measuring one or more attributes of an environment proximal to the trigger device. Said sensors may optionally comprise any one or more of a temperature sensor for measuring ambient temperature or changes in ambient temperature, a light sensor for sensing ambient light or changes in ambient light levels and a sound sensor or recorder, such as a microphone, for sensing ambient noise or changes in ambient noise levels. Optionally the one or more attributes of an environment may comprise any one or more of ambient temperature, changes in ambient temperature, ambient light levels, changes in ambient light levels and ambient noise levels or changes in ambient noise levels.

In some embodiments, the trigger device comprises stimulus means for outputting a stimulus to the user. The stimulus means may, in some embodiments, be disposed in the handle. Advantageously, this enables the device to provide feedback to the user in response to said one or more physiological and/or behavioural parameters of the user.

Optionally, the stimulus means comprises a linear resonant actuator "LRA".

In some embodiments, the ring is releasable connected to the handle. Advantageously, this enables the ring to be worn by a user without having to carry around the handle. This advantageously provides a less intrusive way of sensing said one or more physiological and/or behavioural parameters of the user over a period of time, such as a day, and improves user comfort.

Optionally, the ring is releasably connected to the handle by means of magnets in the ring and the handle. Advantageously, this provides a user friendly way of quickly and easily attaching and detaching the ring to and from the handle, respectively.

In some embodiments, the handle comprises a memory means for storing sensed information. Advantageously, this enables the handle to store information related to sensed said one or more physiological and/or behavioural parameters throughout a period of time, such as a day, a week or a month, for example, for analysis at a later point in time.

In some embodiments, the handle comprises a transmitting means for transmitting sensed information to a remote receiving device. Advantageously, this enables information relating to said one or more physiological and/or behavioural parameters to be collected by the trigger device and transmitted to another device for analysis or data storage. This lightens demand on any processing means, such as a processor, which may be provided in the handle. The transmitting means also advantageously enables a more compact trigger device to be provided, improving portability and user comfort when using the trigger device.

According to still another aspect of the disclosure, there is provided an apparatus for inducing sleep, the apparatus comprising:
stimulus means for providing a user with a first stimulus
input means for providing information relating to the user in response to the stimulus;
processing means for processing information relating to a user provided by said input means, to determine a characteristic of said information; and
a base unit housing one or more of said means,
wherein the apparatus is arranged to sequentially provide the user with a plurality of subsequent stimuli, each subsequent stimulus being based on said characteristic determined from the information provided in response to a previous stimulus,
and wherein the input means comprises the ring of any one or combination of the embodiments described above, or the trigger device of any one or combination of the embodiments described above.

Advantageously, this provides an improved apparatus for inducing sleep.

In some embodiments, of the apparatus,
the input means is arranged to provide historic information relating one or both of: one or more sensed physiological and/or behavioural parameters of the user and one or more sensed attributes of an environment proximal to the input means;
the processing means is arranged to process said historic information to determine one or more characteristics of said historic information; and
at least the first stimulus is determined in dependence on said one or more characteristics of said historic information.

Optionally, said historic information comprises information obtained over the 24 hours preceding operation of the apparatus. Optionally, said historic information comprises information obtained over the 7 days preceding operation of the apparatus. Optionally, said historic information comprises information obtained over the month preceding operation of the apparatus. Optionally, said historic information comprises information obtained over the year preceding operation of the apparatus.

In an embodiment, the stimulus means is a display and said stimulus is light. The display can be mounted on said base unit and selectively positionable, in use, into a field of view of a subject.

Preferably, a position and/or brightness and/or a colour and/or a shape of the next stimulus on the display and/or the time interval between stimuli is based on the characteristic of the active input provided in response to a previous stimulus. As the subject becomes less alert, the delay time between individual light stimuli could be increased. Eventually, as the subject ceases to respond actively to the apparatus, no further light stimuli are provided and the apparatus may switch itself off.

In an embodiment, the display comprises an upper display and a lower display. The upper display and/or said lower display may each comprise an elongate track, preferably oriented generally horizontally when in the field of view of a subject. Each elongate track may be mounted on a support arm attached to said base unit, each elongate track being vertically separated from the other.

In an embodiment, said processing means determines whether said next stimulus should be displayed on the upper or the lower display depending on said characteristic of said active input. Preferably, at least the first of said stimuli is displayed on said upper display.

In an embodiment, said stimuli comprise a sequence of lights moving horizontally from left to right across the subject's field of view.

In an alternative embodiment, said stimulus means is an audio transducer and said stimulus is sound. The audio transducer may be, for example, a loudspeaker, headphones or earphones. Preferably, a volume and/or pitch and/or timbre of the next sound stimulus and/or the delay time between sound stimuli is based on the characteristic of the active input provided in response to a previous sound stimulus.

For example, the volume and pitch may be decreased as the subject becomes less alert, or the timbre changed to a softer sound. As the subject becomes less alert, the delay time between individual sound stimuli could be increased. Eventually, as the subject ceases to respond actively to the apparatus, no further sound stimuli are provided and the apparatus may switch itself off.

In an embodiment, said active input comprises the actuation of a switch, for example the pressing of a button. The characteristic of said active input may comprise a time delay between the provision of the stimulus to the subject and the actuation of the switch by the subject.

In an embodiment, the display of said next stimulus transitions from said upper display to said lower display in response to an increase in said time delay.

The apparatus may further comprise monitoring means for recording a physiological characteristic and/or a behavioural characteristic of the subject, wherein said next stimulus is based on a characteristic of said active input and said physiological and/or behavioural characteristic. The physiological characteristic may comprise, for example, any one or more of, or any combination of one or more of: heart rate, body temperature, skin temperature, galvanic skin response, blood oxygen saturation levels (SpO2), mixed venous oxygen saturation (SvO2), exerted grip force, reaction time or times, respiratory rate, blood pressure, movement, muscle contraction and/or relaxation, a composition or at least one or more constituents of exhaled air, electrical brain activity, body temperature, blood pressure and respiratory rate.

The apparatus may further comprise ambient monitoring means for recording an ambient characteristic of the subject's environment, wherein said next stimulus is based on characteristic of said active input and said ambient characteristic. The ambient characteristic may be one or more of, for example, time, temperature, light level and sound level.

In an embodiment, the determination of said next stimulus is additionally based on a characteristic of at least one active input provided in response to a stimulus provided before the immediately preceding stimulus.

According to another aspect of the disclosure, there is provided apparatus for inducing sleep comprising:
a display for providing a subject with a light stimulus;
a base unit on which said display is mounted such that the display is selectively positionable, in use, into a field of view of a subject;
input means, for recording an active input provided by the subject in response to the stimulus;
processing means for processing said active input to determine a characteristic of said active input; and
a base unit housing one or more of said means,
wherein the apparatus is capable of sequentially providing the subject with a plurality of stimuli, each next stimulus being based on a characteristic determined from the active input made in response to a previous stimulus, and wherein a position and/or brightness and/or a colour and/or a shape of the next stimulus on the display and/or a time interval between stimuli is based on the characteristic of the active input provided in response to a previous stimulus,
and wherein the input means comprises the ring of any one or combination of the embodiments described above, or the trigger device of any one or combination of the embodiments described above.

According to another aspect of the disclosure there is provided a method for inducing sleep comprising:
providing a subject with an initial stimulus;
recording an active input provided by the subject in response to the initial stimulus;
processing said active input to determine a characteristic of said input;
providing the subject with a further stimulus based on said characteristic;
recording a further active input provided by the subject in response to the further stimulus;
processing the further active input provided by the subject in response to the second stimulus to determine a further characteristic; and
sequentially providing the subject with a plurality of further stimuli, each further stimulus being based on a characteristic determined from the active input made in response to the previous stimulus.

The method can be performed using the apparatus of any of the preceding paragraphs.

According to yet another aspect of the invention, there is provided a kit comprising:
the ring of any one or combination of embodiments as above described; and
one or more replacement pads.

Advantageously, this enables the pad to be removed and cleaned, or replaced with a clean pad, thereby improving hygiene of the ring.

Optionally, the one or more replacement pads comprise different dimensions relative to each other. This enables the replacement of the pad, for example with a pad of different dimensions. This enables the ring to be used by a variety of users having any variety of finger shapes or sizes.

According to another aspect of the disclosure, there is provided a kit comprising:
the trigger device of any one or combination of embodiments as above described; and
one or more replacement pads.

Advantageously, this enables the pad to be removed and cleaned, or replaced with a clean pad, thereby improving hygiene of the trigger device.

Optionally, the one or more replacement pads comprise different dimensions relative to each other. This enables the replacement of the pad, for example with a pad of different dimensions. This enables the trigger device to be used by a variety of users having any variety of finger shapes or sizes.

According to still another aspect of the disclosure, there is provided a kit comprising:
the apparatus of any one or combination of embodiments as above described; and
one or more replacement pads.

Advantageously, this enables the pad to be removed and cleaned, or replaced with a clean pad, thereby improving hygiene of the apparatus.

Optionally, the one or more replacement pads comprise different dimensions relative to each other. This enables the replacement of the pad, for example with a pad of different dimensions. This enables the apparatus to be used by a variety of users having any variety of finger shapes or sizes.

### BRIEF DESCRIPTION OF THE DRAWINGS

One or more embodiments of the invention will now be described by way of example only, with reference to the accompanying drawings, in which:
Figure 1 shows a schematic section through a human finger and a number of relative positions about a circumference of the finger;
Figure 2 shows a ring according to an embodiment of the invention;
Figure 3A shows a partial side view of a trigger unit ;
Figure 3B shows a partial side view of another trigger unit ;
Figure 3C shows a partial side view of yet another trigger unit;
Figure 4A shows a perspective view of a trigger unit with a resiliently deformable pad removed from a ring of the trigger unit,
Figure 4B shows a cross sectional view of part of a trigger unit;
Figure 4C shows a perspective view of a ring of a trigger unit;
Figures 5A to 5C show side views of trigger units, each unit having a resiliently deformable
pad of differing dimensions,
Figures 6A to 6H show side views of trigger units, each unit having a resiliently deformable pad of differing dimensions,
Figure 7A shows a top perspective view of a trigger unit;
Figure 7B shows a partial cross sectional view of a trigger unit with the resiliently deformable pad omitted for clarity;
Figures 8A and 8B show another trigger unit; and
Figure 9 shows an apparatus for inducing sleep.

With reference to the Figure 2, embodiments of the invention relate to a ring 200 for sensing one or more physiological and/or behavioural parameters of a user. The ring 200 comprises an aperture 202 for receiving a finger of the user therethrough. The aperture 202 comprises a sensing portion, which, in at least the illustrated embodiments, comprises a substantially V-shaped portion 204 and one or more sensing means 206A, 206B, 206C, 206d, 206e disposed in the sensing portion. The sensing means 206A - 206e may comprise any one or more of a temperature sensor 206e for sensing a temperature of the finger or of the skin of the finger, a galvanic skin response "GSR" sensor 205A, 205B for sensing an electrical conductance of the finger or of the skin of the finger, an optical sensor 205C, 206d for sensing heart rate of the user, an accelerometer for sensing movement of a user, a gyroscope for sensing an orientation of the ring 200 and a force sensor for sensing a pressure applied by the finger against the sensing portion, which, as shown in Figure 2, comprises a substantially V-shaped portion 204. It will be appreciated by the reader that this list is by no means exhaustive and a variety of other sensing means may be used within the scope of the invention.

The aperture 202 of the ring 200 comprises a resiliently deformable pad 208, opposing the sensing portion, in some embodiments, an apex 209 of the substantially V-shaped portion 204 of the sensing portion. The resiliently deformable pad 208 may comprise silicone rubber, although it will be appreciated that other elastomers and resiliently deformable materials may be used. Alternatively, the pad may be made from a non-resilient or substantially rigid material. In some embodiments, the resiliently deformable pad 208 comprises a plurality of projections 210 for contacting the finger.

Figure 1 shows a schematic cross section through a human finger 100, which may be an index finger, and a number of positions 151, 152, 153, 154, 155 for clarifying an orientation of the finger 100. Position 1, 151 indicates a side of the finger 100 proximal to an adjacent thumb (not shown), whilst position 5, 155 indicates an opposing side of the finger 100, distal to the thumb. Position 3, 153 indicates a central position on an underside 160 of the finger 100. As shown in Figure 1, the underside 160 may be differentiated from an opposing topside 170 of the finger 100 in that a bone 102 of the finger lies proximal to skin 110 of the topside 170, with relatively little to no fat 106 between the skin 110 and the bone 102. Contrastingly, at the underside, fat 106 and one or more ligaments 104 may interpose the skin 110 and the bone 102. Position 2, 152 indicates a position on the underside 160 intermediate central position 153 and side position 151, proximal to the thumb. Position 4, 154 indicates a position on the underside 160 intermediate central position 153 and side position 155, distal to the thumb.

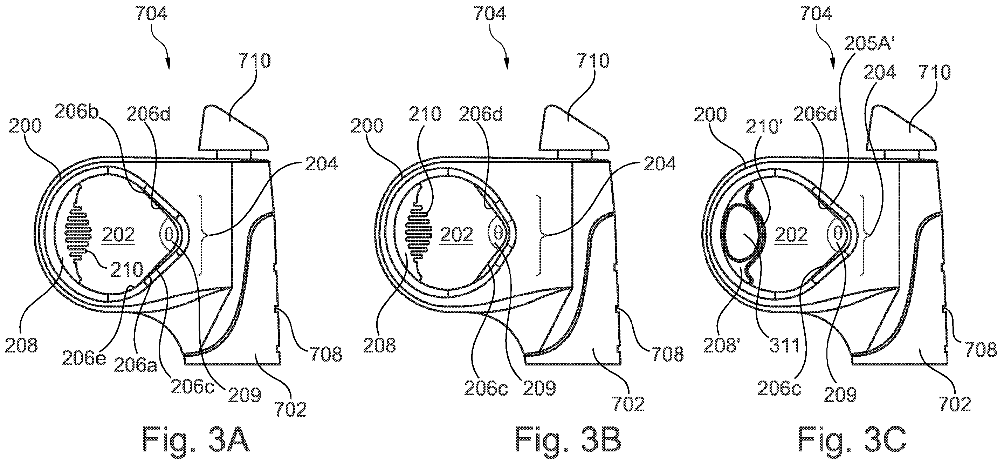

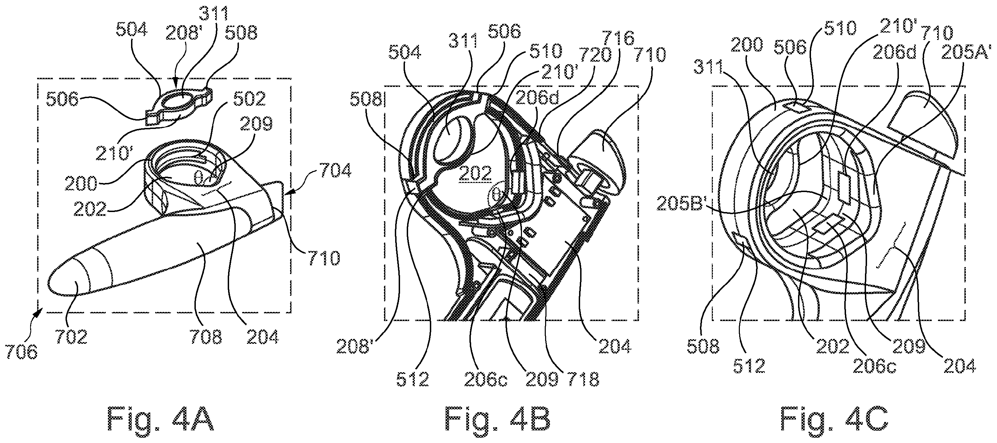

Referring again to Figure 2, in use, the aperture 202 is arranged to receive a finger 100 therein, such that central position 153 on the underside 160 of the finger 100 is substantially aligned with the sensing portion of the aperture 202 of the ring 200. In embodiments in which the sensing portion comprises a substantially V-shaped portion 204, as shown in the Figures, the aperture 202 is arranged to receive a finger 100 therein, such that central position 153 on the underside 160 of the finger 100 is substantially aligned with the apex 209 of the substantially V-shaped portion 204. In some embodiments, the aperture 202 is additionally arranged to receive a finger 100 such that a joint of the finger, preferably a proximal interphalangeal "PIP" joint of the finger is aligned with the ring 200. Preferably the finger 100 is an index finger. The resiliently deformable pad 208 is arranged to contact at least a part of the topside 170 of the finger 100 to urge the finger 100 against at least one of the one or more sensing means 205A, 205B, 205C, 206d, 206e when the finger 100 is received in the aperture. In some embodiments, such as that shown in Figures 3C, 4C, 7A and 7B, one or more of the sensing means, such as a GSR 205A', 205B' may be provided over an area spanning at least a length of the sensing portion, such as the substantially V-shaped portion 204. Figure 4C shows two GSR sensors 205A', 205B', each covering a substantially V-shaped portion 204, proximal to and extending beyond a threshold of the aperture. Although the GSR sensors 205A', 205B' shown in the Figures as following the substantially V-shaped portion 204, it will be appreciated that the sensors 205A', 205B' may cover any area shape, as long as they are not in direct contact with other sensing means, such as the optical sensors 206c, 206d. In some embodiments, the GSR sensors 205A', 205B' may cover an area of between 50mm² and 109mm².

Turning now to Figures 3A to 3C, it will be appreciated that the apex 209 of the aperture 202 may comprise any one of a variety of angles θ. However, according to the invention, the angle θ comprises between 85° and 120°. In the embodiment shown in Figure 3A, the angle θ comprises 85°, whilst in the embodiment shown in Figure 3B, the angle θ comprises 120°. In the embodiment shown in Figure 3C, the angle θ comprises 105°.

In some embodiments, the resiliently deformable pad 208 may be removable. With reference to the embodiments illustrated in Figures 4A to 4C, the pad 208 is receivable in a slot 502 in the ring 200. The pad 208 may be releasably retained in the ring 200 by any number of conventional fastenings or mechanical fixings. In the embodiment shown in Figures 4A to 4C, the pad comprises a body portion 504 and a pair of pips 506, 508 either side of the body 504. The pips 506, 508 are arranged to cooperate with female detents 510, 512 in the ring 200.

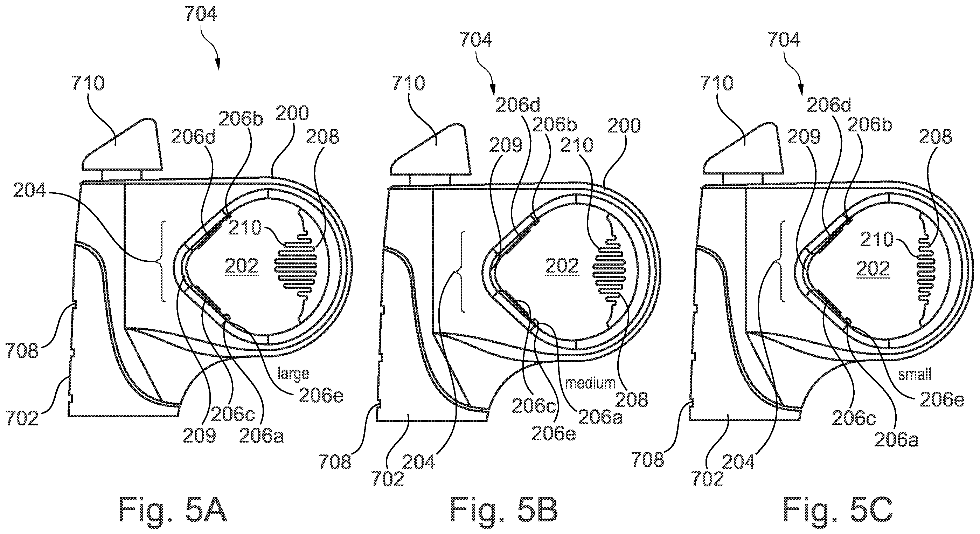

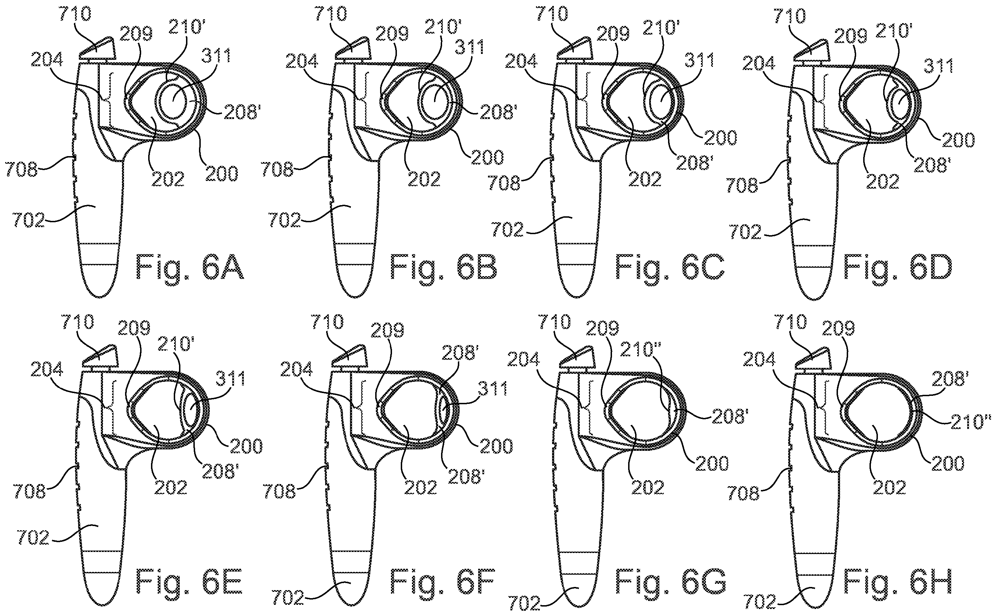

With reference to Figures 5A to 5C and 6A to 6H, the resiliently deformable pad 208 may comprise a variety of sizes. Figures 5A to 5C show rings 100 comprising a relatively large pad 208, a relatively medium pad 208 and a relatively small pad 208, respectively. The relative sizes of the pads 208 are increased or decreased by varying the length of the part of the pad 208 which extends into the aperture 202. In the embodiments shown in Figures 5A to 5C, the relative sizes of the pads 208 are increased or decreased by varying the length of the projections 210 of the pad 210. However, it will be appreciated that the sizes may vary by additionally or alternatively varying other dimensions of all of the pad 204, or of only portions, such as the body 504, of the pad 208.

With reference to Figure 3C and Figures 6A to 6H, the resiliently deformable pad 208' may comprise a concave or a convex surface 210", 210' and optionally a through hole 311. Figures 6A to 6H illustrate an embodiment in which rings 100 comprise a pads 208' for users having gradually increasing relative finger diameters. A relatively small finger diameter requires a resiliently deformable pad 208' having a relatively convex surface 210', such as that shown in Figures 6A to 6D, whilst a slightly larger finger diameter may require a pad 208' having a relatively less convex surface 210', as shown in Figures 6E and 6F. A relatively large finger diameter may require a pad 208' having a relatively concave surface 210".

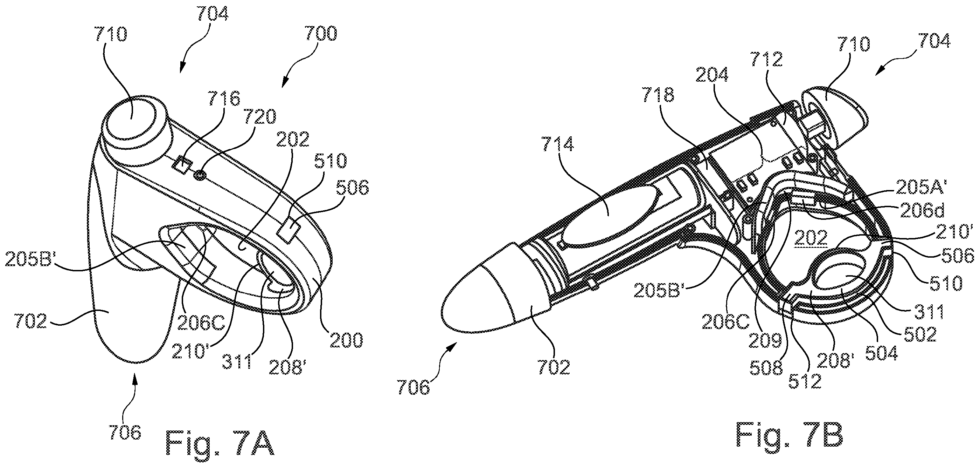

Figures 7A and 7B show a trigger device.

The trigger device 700 may be a handheld trigger device and may be portable. The trigger device 700 comprises a handle 702 for gripping by a user. The handle 702 comprises a proximal end 704 and a distal end 706. The trigger device 700 comprises a ring 200, such as any one of or any combination of the embodiments of the ring 200 as above described. The ring 200 extends laterally or radially from the proximal end 704 of the handle 702.

At least a part of the handle 702 may comprise a textured surface 708, as shown in Figures 3A, 3B, 4A, 5A to 5C, 6A to 6H and 8A and 8B, for facilitating gripping of the handle by the user. The handle 702 may comprise a user input button 710 disposed at the proximal end 704 of the handle 702. The user input button 710 is arranged for pressing, for example, by a thumb of a user and may comprise a force sensor 712. In addition or alternatively to the user input button 710, the handle 702 may comprise one or more means for sensing other user input(s). In the embodiment shown in Figure 7B, the handle 702 comprises both the user input button 710 connected to the force sensor 712 and a grip force sensor 714 for sensing a force with which the user grips the handle 702.

The trigger device 700 may comprise one or more sensors 716 for measuring one or more environmental attributes proximal to the trigger device, such as: duration of exposure to natural daylight and/or synthetic light, noise levels, ambient temperature. The one or more sensors may comprise any one or more of a temperature sensor 716, a light sensor and a sound sensor, such as a microphone. The one or more sensors may be disposed in one or both of the ring 200 and the handle 702.

In some embodiments, the trigger device 700 comprises means, such as a linear resonant actuator "LRA" 718, for outputting a stimulus, such as a sequence of vibrations via the handle 702, to a user. The trigger device 700 may comprise a processing means, such as a processor (not shown) for determining an appropriate output stimulus, such as a particular sequence of vibrations in response to said one or more sensed physiological and/or behavioural parameters of the user, one or more environmental attributes proximal to the trigger device, and an active user input, such as a rate or force with which the user presses the user input button 710 and/or a force with which the user grips the handle 702 of the trigger device. In this way, the stimulus may be varied in dependence on a user's environment and/or one or more physiological and/or behavioural parameters of the user.

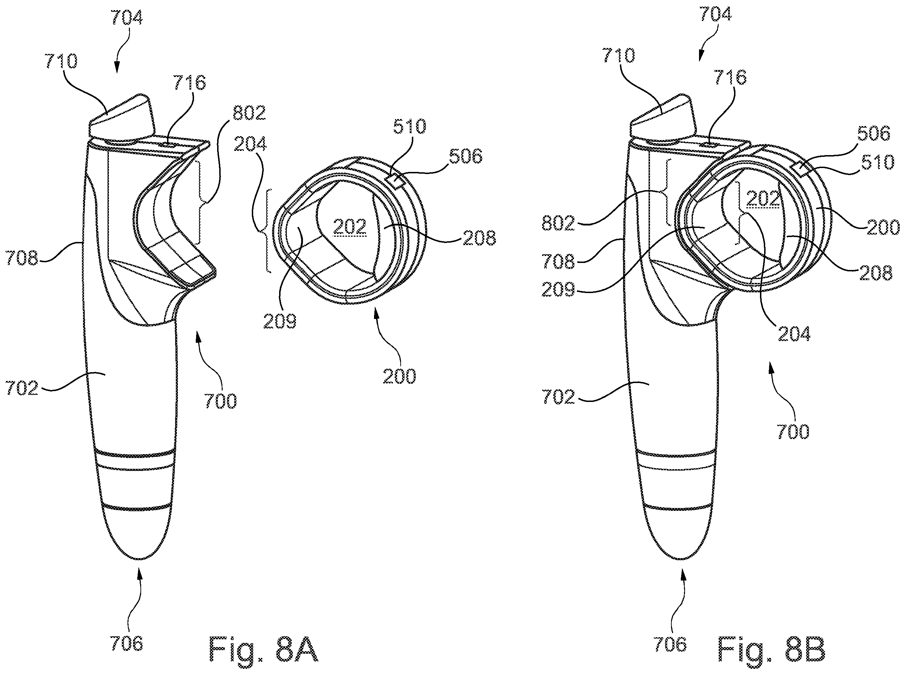

Referring now to Figures 8A and 8B, the ring 200 may be releasably connected, such as by means of a magnetic engagement, to the handle 702. In the embodiment illustrated in Figures 8A and 8B, the ring 200 is releasably connected to the handle 702 by means of one or more magnets disposed in the sensing portion, which may comprise a substantially V-shaped portion 204, of the aperture 202 of the ring 200 and in a correspondingly V-shaped portion 802 of the handle 702. In this way, a user may choose to use only the ring 200 for the majority of the day, to non-intrusively collect data, before later connecting the ring 200 to the trigger device 700. The trigger device 700 may then output an appropriate stimulus, as above described. Alternatively, the ring 200 may itself be capable of outputting an appropriate stimulus.

The ring 200 may comprise a memory means, such as a non-transitory memory for storing information, collected throughout a period of time such as a day, relating to sensed physiological and/or behavioural parameters of the user and optionally, information relating to one or more environmental attributes proximal to the ring 200. The trigger device 700 may comprise data transfer means for downloading or at least retrieving data from the memory means of the ring 200 when the ring 200 is connected to the handle 702. In this way, a user may receive an appropriate stimulus, at the end of a period, such as a day, without having to carry around the entire trigger device 700 with them for the entire period.

In some embodiments, one or both of the ring 200 and the handle 702 comprise means for transmitting information to a remote receiving device, such as a computer, an electronic device or a portable electronic device. The means for transmitting information may comprise means for wirelessly transmitting information, such as Bluetooth^{®}.

According to another aspect of the disclosure, the ring 200 or the trigger device 700 may be incorporated as an input means as part of an apparatus for inducing sleep in a subject. A subject consciously thinking about problems and worries may find it difficult to fall asleep. The apparatus and methods create a distraction for the conscious mind to help the subject rest and relax, inducing sleep more successfully. The apparatus and methods of the present disclosure encourage and use distraction techniques such as meditation and trigger based cognitive therapy to help the subject unwind and fall asleep.

Cognitive therapy helps create new neural pathways through habit-forming patterns using anchors/triggers. After repeated use, the mere switching on of the apparatus when the subject goes to bed may be enough of an anchor in itself to instruct the mind to relax. Operation of the apparatus as described below provides patterns of stimuli to the subject to distract them from conscious thoughts and worries which might otherwise deter them from falling asleep, thus reducing the time taken to induce sleep. The stimuli are tailored to the subject by responding to active input by the subject, as described below.

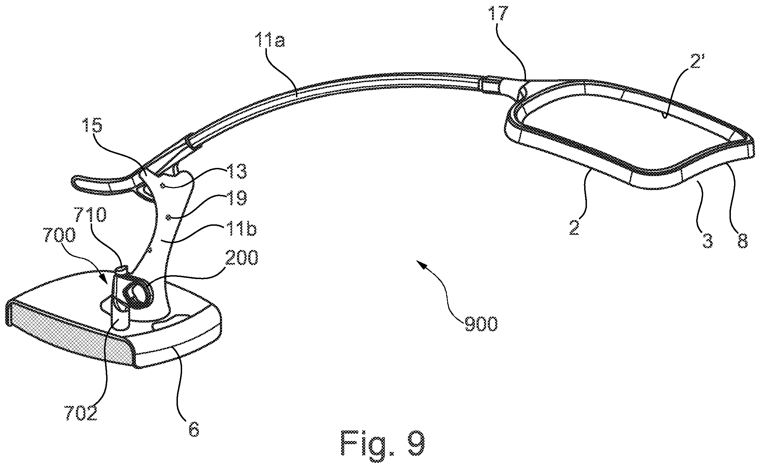

Figure 9 shows an apparatus for inducing sleep.

The apparatus 900 includes stimulus means, which in the illustrated embodiment comprises upper and lower displays in the form of elongate tracks 2 and 2' provided on a head 3. Each track has plurality of lights, for example LEDs, disposed thereon and the upper and lower tracks are vertically separated from each other. The elongate tracks may be curved.

The tracks 2, 2' are mounted on support arms 11a, 11b connected to a base unit 6. The base unit 6 may take the size and form of a typical bedside clock radio unit and is intended to be normally located on a bedside table or the like so that it is readily accessible for use every night. Alternatively, the base unit 6 may be incorporated into the headboard of a bed or provided as a wall or ceiling unit, mountable above or near the bed. Other alternatives may be envisaged. The tracks and support arms are preferably pivotally connectable, such as by means of a hinge 13, to the base unit 6 so that the tracks 2, 2' are selectively positionable into a field of view of the subject once the subject is laying down in bed and can be pivoted up and out of the way (over or into the base unit) when not required. A height of the tracks and support arms is preferably adjustable, such as by means of height adjuster 15. Preferably the tracks 2, 2' are orientated generally horizontally when in the field of view of the subject.

The apparatus 900 further comprises input means (for example the trigger device 700) for measuring an active (conscious) input provided by the subject and monitoring means for measuring a physiological characteristic and/or behavioural characteristic of the subject. The monitoring means may be provided as part of the trigger device 700 or ring 200, or by another device (not shown). Processing means comprising control hardware and software (not shown) may conveniently be disposed within the base unit 6. Both the input means and the monitoring means are also connected to or are part of the base unit 6.

When the subject is ready to go to sleep, they lay in bed with the tracks 2, 2' above their head in their field of view. The upper display, i.e. track 2 may be positioned so that it is slightly uncomfortable for the subject to view, whereas the lower display, i.e. track 2' may be positioned in an ideally comfortable viewing position. The subject holds the trigger device 700 in one hand by gripping the handle 702 of the trigger device 700, with their index finger inserted through the aperture 202 of the ring 200 such that a central 153 underside 160 of the finger and a PIP joint of the index finger is substantially aligned with the apex 209 of the substantially V-shaped portion 204 of the aperture 202 of the ring 200.

The apparatus 900 is configured to provide a subject with a visual stimulus by illuminating at least one of the lights on the elongate track 2 or 2'. The initial stimulus may be the illumination of the leftmost LED on the upper track 2. When the subject sees the initial stimulus, he/she actuates the input means (e.g. by pressing the user input button 710 of the trigger 700) so that the apparatus 900 receives an active input from the user made in response to the visual stimulus.

When the apparatus receives an active input from input means, the control hardware is configured to record and process the input to determine at least one characteristic of the input. The control hardware is then configured to calculate and provide a second stimulus to the subject, which stimulus is provided by one of the lights on tracks 2 or 2'. For example, the second stimulus may be the illumination of the second from left LED on the upper track 2. The determination of said second stimulus is at least partially based on the determined characteristic of the initial input.

A second active input provided in response to the second stimulus is then provided by the subject. The second active input is recorded by the control hardware and a characteristic of that input is determined. Subsequent stimuli are then sequentially provided, with the calculation of each subsequent stimulus being based at least partially on the determined characteristics of the inputs made in response to a previous stimulus. "Previous stimulus" means the immediately preceding stimulus and/or earlier stimuli.

For example, a sequence of stimuli might comprise the sequential illumination of LEDs from left to right, one at a time, in a repeated pattern in response to the subject's active inputs. The speed at which the pattern progresses, i.e. the time delay between each stimulus could depend on the subject's speed of response. When the subject is most alert, there may be little time delay between the creation of the stimulus and the subject's active response. As the subject becomes less alert and sleepier, the subject will respond more slowly. The apparatus can detect this and make appropriate changes to the pattern or timing or other characteristic of the next stimuli to maximise the meditative and/or cognitive effect to encourage sleep.

For example, the brightness of the light stimulus may be decreased, the light stimulus may be provided on the lower of the two curved tracks 2, 2', or a combination of the above changes may be made. Furthermore, a passive input may also be taken from the subject in the form of a monitored physiological and/or behavioural characteristic, such as movement of the subject and/or respiratory rate, which input may be used to detect how close the subject is to falling asleep. The passive input may comprise one or more physiological and/or behavioural parameters of the subject sensed by the sensing means 206a - 206e of the ring 200, and/or one or more environmental attributes proximal to the trigger device 700, such as an ambient temperature sensed by the one or more sensors 716 of the trigger device 700. The passive input may additionally or alternatively comprise one or more environmental attributes sensed by one or more sensors disposed elsewhere on the apparatus 900, such as ambient temperature, humidity and light sensors 17 disposed in arm 11a or the head 3, and/or carbon dioxide and/or barometric pressure sensors 19 disposed in the arm 11a and/or arm 11b, as shown in Figure 9. The stimuli provided to the subject may then be calculated based on both the active and the passive input. The passive input may be any one of, or any combination of said behavioural and/or physiological parameters and/or said one or more environmental attributes.

The calculation of the stimuli may determine any one of, or any combination of, the brightness, colour, shape, position, delay time before illumination, the speed of illumination of the lights disposed on tracks 2 and 2', the track on which the light that is illuminated is disposed and the position on the track of the light that is illuminated.

Active input means may be the user input button 710 itself, which the subject actuates in response to the stimulus, thus providing an active input. As discussed above, said user input button 710 may be disposed on the proximal end 704 of the handle 702 of the trigger device 700. The determined characteristic of the input may be one of, or any combination of, the time delay between the stimulus and the input, the speed with which the user input button 710 is depressed, the pressure applied to the button 710, the length of time for which the button 710 remains pressed, movement or vibration of the input means when the button 710 is pressed and the position of the input means in the subject's hand when the button 710 is pressed.

The determination of the stimuli may additionally be based on a characteristic of a physiological and/or behavioural characteristic of the subject recorded by monitoring means. The monitoring means may comprise a band to be placed around the subject's finger, wrist, chest or another suitable part of the subject's body. Alternatively, the monitoring means may be part of the trigger device 700, such as the sensing means 206a to 206e.

The monitoring means measures a physiological and/or behavioural characteristic of the subject, which may be any one of, or any combination of: heart rate, body temperature, skin temperature, galvanic skin response, blood oxygen saturation levels (SpO2), mixed venous oxygen saturation (SvO2), exerted grip force, reaction time or times, respiratory rate, blood pressure, movement, muscle contraction and/or relaxation, a composition or at least one or more constituents of exhaled air, electrical brain activity, body temperature, blood pressure and respiratory rate, duration of light exposure (which may comprise duration of exposure to natural and duration of exposure to artificial light), exercise and/or activity levels, such as steps taken and/or movement whilst awake, sleep characteristics, such as movement during sleep, snoring, or restless legs. It will be appreciated by the reader that the list of exemplary physiological parameters is not exhaustive.. The monitoring means may continue to measure physiological and/or behavioural characteristics of the subject whilst the subject is asleep, which characteristics may include involuntary behaviour whilst asleep, such as involuntary movement whilst asleep. This data may be usefully used later for conventional sleep analysis.

The apparatus 900, may also include ambient monitoring means (not shown) for recording ambient characteristics of the subject's environment, for example the time, room temperature, light level or sound level. Ambient monitoring means may be provided as part of the trigger device 700, such as the one or more sensors 716.

Optionally the active input means, the monitoring means and/or the ambient monitoring means may all be provided as part of the trigger device 700. Alternatively, the active input means and the monitoring means may be separate devices, connected to the base unit by a wired connection or by a wireless communication protocol, such as Bluetooth^{®} for example.

It will be readily apparent to the skilled person that the calculation of the stimuli may be performed by conventional control hardware provided with suitable software to calculate the stimuli required in response to a given active input or combination of inputs from the subject (possibly in combination with physiological and/or behavioural and/or ambient data). Such control hardware may comprise a processor configured to receive electrical signals from the active input and the monitoring means, process the data provided thereby and provide an output signal to the lights. Software implementing predetermined algorithms may be used to adjust the output provided to the lights based on the received inputs and determined characteristics. The software may reduce or increase the length of time between stimuli, the brightness of the lights and the location of the activated lights based on the characteristic of the inputs made in response to the stimuli. In one embodiment the software may provide light stimuli which move horizontally, either from left to right or right to left, across the subject's field of vision, and the delay time between stimuli may be adjusted based on a characteristic of the subject's response. The characteristic of the subject's response may be the delay time between the provision of a stimulus and the provision of an active input in response to the stimulus.

Furthermore, as the subject becomes more tired and the delay time between the provision of the stimuli and the provision of the active inputs in response to the stimuli increases, the software may activate the lights on the lower track 2' rather than the upper track 2. The transition of the stimuli from the upper track 2 (which is relatively uncomfortable to view) to the lower track 2' (whose position is ideally comfortable to view) may in itself have the effect of encouraging the subject to close their eyes.

The software may implement machine learning techniques to optimise the stimuli provided to the subject over time. Such machine learning may comprise detecting when the subject has fallen asleep, recording the pattern of stimuli that were delivered before the subject fell asleep and adjusting future stimuli based on the recorded patterns of stimuli.

The control hardware may conveniently be disposed within the base unit 6, and the inputs and outputs may be received by and sent from the control hardware either via direct electrical connections or wirelessly using conventional wireless communication technology. If wireless communication is employed between the input means and monitoring means and/or the lights then the input means and/or the lights may be physically connected to the base unit 6 for charging when the apparatus is not in use.

In the embodiment shown in Figure 9, the display comprises LED lights provided on tracks 2, 2'. Alternatively, the display may comprise one or more screens or other devices capable of displaying light at different locations.

The stimulus may alternatively or additionally be audible rather than visual, as shown in Figure 9. In this embodiment the stimulus means is an audio transducer. In the illustrated embodiment the audio transducer is a loudspeaker 8, which is shown disposed on the display. In other embodiments the audio transducer may be headphones or earphones or the like, or may be disposed on the base unit 6.

The stimuli provided by loudspeaker 8 are calculated based on a characteristic of at least one active input provided by the user, and optionally also based on data from the monitoring means and/or ambient monitoring means 17, 19.

The calculation of the audible stimulus may comprise adjusting any one of, or any combination of, the volume, pitch or timbre of the stimulus and/or delay time between stimuli.

The apparatus 900 may be operated in an "audible only" mode wherein only the loudspeaker provides stimuli, a "visual only" mode wherein only the lights provide stimuli or a "combined" mode wherein both the lights and the loudspeaker provide stimulus. This embodiment may provide an apparatus suitable for use by people who are either blind or deaf.

The skilled reader will appreciate that stimuli other than sound and light may also be used. For example, a vibration or other tactile stimulus may be provided via the trigger device 700 a worn wristband or the like.

Although the above apparatus has been described with respect to use in inducing sleep, it will be understood that additional uses are also possible. For example, the apparatus 900 or indeed the trigger device 700 alone may be used for practicing mindfulness. Such additional uses may include (without limitation), measuring the alertness of a subject, helping to calm an anxious subject, helping a subject to wake up from sleep and a gaming mode. Furthermore, additional functions may conveniently be incorporated into apparatus 900. Such additional functions may include (without limitation) an alarm clock, a radio, a SAD light, a docking station for a mobile phone or music player, a CD player, and a measurement device to record the ambient characteristics of the subject's environment, these most likely but not necessarily being located in the base unit 6. The ambient characteristics that the measurement device is configured to measure may include (without limitation) one or more of time, room temperature, light level and sound level.

In a further embodiment, the base unit 6 may have USB/internet ports for additional inputs providing contemporaneous physiological and/or behavioural data, for example EEG, which could contribute to the calculation of the stimuli. USB or wireless inputs could also be used to download historic physiological and/or behavioural data to the apparatus, for example data from a step counter collected during the previous day, so that the apparatus can take into account the subject's level of daily physical activity when calculating the stimuli. The base unit 6 may include means to enable a smartphone to plug in directly in order to download such data.

In embodiments having an audio stimulus, the apparatus 900 could be programmed to include an audible or subliminal command to the subject to close their eyes at an appropriate stage (for example as a light stimulus transitions from the upper display to a lower display). Other words or commands, such as those that might typically be used in hypnosis (e.g. "relax", "notice the lights dim", "let your eyelids close" etc) may be incorporated into an audio output.

The apparatus 900 could take the form of an all-in-one bedroom sleep inducing and monitoring tool including, not only the sleep inducing function described above but also more conventional physiological and/or behavioural and optionally ambient data collection and analysis capability.

In some embodiments of the apparatus 900, the trigger device 700 may be portable and connectable with the remainder of the apparatus 900 as required, such as when using the apparatus 900 to induce sleep. The trigger device 700 may comprise a memory means (not shown), such as a non-transitory memory for storing information, collected throughout a period of time such as a day, relating to sensed physiological and/or behavioural parameters of the user and optionally, information relating to one or more environmental attributes proximal to the trigger device 700. The trigger device 700 may be connectable, for example to the base unit 6 by a wired connection. Alternatively, the trigger device 700 may be connectable to the remainder of the apparatus 900 by means of a wireless communication protocol, such as Bluetooth ^{®}. In this way, the user may carry around the trigger device 700 throughout a period, such as a day, and connect the device to the apparatus 900 when they are ready to start using the apparatus 900. The apparatus 900 may then provide the stimulus or stimuli in dependence on one or more physiological and/or behavioural parameters of a user and optionally one or more environmental attributes proximal to the trigger device 700 throughout the day.

In embodiments wherein the ring 200 of the trigger device 700 of the apparatus 900 is releasably connected to the handle 702 of the trigger device 700, as described above in relation to embodiments of the trigger device 700 in particular, the ring 200 may comprise a memory means (not shown). The memory means may comprise a non-transitory memory for storing information, collected throughout a period of time such as a day, relating to sensed physiological and/or behavioural parameters of the user and optionally, information relating to one or more environmental attributes proximal to the ring 200. The ring 200 may be connectable, for example to the handle 702 of the trigger device 700 or indeed, directly to the base unit 6, by a wired connection. Alternatively, the ring 200 may be connectable to the remainder of the apparatus 900 by means of a wireless communication protocol, such as Bluetooth ^{®}. In this way, the user may carry around the ring 200 throughout a period, such as a day, and connect the ring 200 to the apparatus 900 when they are ready to start using the apparatus 900. The apparatus 900 may then provide the stimulus or stimuli in dependence on one or more physiological and/or behavioural parameters of a user and optionally one or more environmental attributes proximal to the ring 200 throughout the day.

The trigger device 700 may comprise one or more LEDs 720. The one or more LEDs 720 may provide a user with an indication as to one or more operative characteristics of the trigger device 700. Such characteristics may comprise whether the device is switched on, whether a finger of the user is received in the aperture 202 in a sufficient fit and orientation to sense said one or more physiological and/or behavioural parameters of the user, whether the device 700 is charging or fully charged, whether the device 700 is synching with a remote device, such as a computer, a portable electronic device or an apparatus for inducing sleep as described above. The one or more operative characteristics may be communicated by means of any of: flashes or pulses of light emitted from the LED, continuous light emitted from the LED, a colour or alternating or changing colour of light emitted from the LED. It will be appreciated the ring 200 may itself may be provided with one or more LEDs as above described, for example, in embodiments wherein the ring 200 is provided alone, without the handle.

Although the description describes use of the ring 200, trigger device 700 and/or apparatus 900 with respect to monitoring and/or inducing sleep, it will be appreciated that the disclosure may be used for sensing physiological and/or behavioural parameters for a variety of different contexts, such as monitoring biomarkers, monitoring a user's vital signs, and/or monitoring a user's clinical presentations, such as stress, anxiety, ageing and general wellbeing, for example.

Throughout the description and claims of this specification, the phrase "**physiological parameter**" and variations thereof mean any physical, biological, anatomical, medical or physiological, characteristic, or combination thereof, of the user. The physiological parameter may comprise, for example, any one or more of, or any combination of one or more of: heart rate, body temperature, skin temperature, galvanic skin response, blood oxygen saturation levels (SpO2), mixed venous oxygen saturation (SvO2), exerted grip force, reaction time or times, respiratory rate, blood pressure, movement, muscle contraction and/or relaxation, a composition or at least one or more constituents of exhaled air and electrical brain activity. It will be appreciated by the reader that the list of exemplary physiological parameters is not exhaustive.

Throughout the description and claims of this specification, the phrase "**behavioural parameter**" and variations thereof may comprise one or more voluntary behavioural parameters, and/or one or more involuntary behavioural parameters, or any combination thereof. The behavioural parameter may comprise any one or more of, or any combination of one or more of: duration of light exposure (which may comprise duration of exposure to natural and duration of exposure to artificial light), exercise and/or activity levels, such as steps taken and/or movement whilst awake, sleep characteristics, such as sleep duration, times at which a user sleeps and is awake, phases of sleep, such as rapid eye movement (REM), deep sleep, light sleep, movement during sleep, snoring, or restless legs. It will be appreciated by the reader that the list of exemplary behavioural parameters is not exhaustive.

Throughout the description and the claims of this specification, the phrase "**environmental attribute**" and variations thereof may comprise any one or more characteristics of an environment proximal to the ring. The environmental attribute may comprise any one or more of, or any combination of any one or more of: humidity, levels of light, levels of natural light, levels of unnatural or artificial light, temperature local to the said ring, trigger device or apparatus, ambient room temperature, pollution levels, oxygen levels and air pressure. It will be appreciated by the reader that the list of exemplary environmental parameters is not exhaustive.

Throughout the description and claims of this specification, the phrase "active input" and variations thereof mean an input which the subject makes a conscious decision to provide, for example the pressing of a button.

Throughout the description of this specification, the phrase "passive input" and variations thereof mean an input which is provided without the subject's conscious decision, for example an input based on a monitored physiological and/or behavioural characteristic, as above described, and/or an input based on a monitored ambient characteristic or environmental attribute, such as room temperature.

Throughout the description of this specification the phrase "inducing sleep" means providing stimuli designed to encourage a subject to fall asleep, and is not intended to (and does not) imply that such stimuli must necessarily be successful in causing the subject to fall asleep.

Throughout the description of this specification, the term "proximal end of the handle" means the end of the handle nearest to the ring. The ring may be located at any point between a central point of the handle and the proximal end extremity or tip. The term "extends radially from the proximal end of the handle" may refer to the ring extending from the handle along an axis which intersects a longitudinal axis of the handle, the ring extending from the handle at any position around a circumference of the handle. The input button may be located in any position at or near the proximal end extremity or tip of the handle, wherein the user can press the input button with a thumb whilst an adjacent index finger is inserted through the ring.

It will be appreciated that embodiments of the present disclosure can be realised in the form of hardware, software or a combination of hardware and software. Any such software may be stored in the form of volatile or non-volatile storage such as, for example, a storage device like a ROM, whether erasable or rewritable or not, or in the form of memory such as, for example, RAM, memory chips, device or integrated circuits or on an optically or magnetically readable medium such as, for example, a CD, DVD, magnetic disk or magnetic tape. It will be appreciated that the storage devices and storage media are embodiments of machine-readable storage that are suitable for storing a program or programs that, when executed, implement embodiments of the present disclosure.

Accordingly, embodiments provide a program comprising code for implementing a system or method of the present disclosure and a machine readable storage storing such a program. Still further, embodiments of the present disclosure may be conveyed electronically via any medium such as a communication signal carried over a wired or wireless connection and embodiments suitably encompass the same.

## Claims

1. A ring (200) for sensing one or more physiological and/or behavioural parameters of a user, the ring (200) comprising:
an aperture (202) for receiving a finger (100) of the user therethrough, the aperture (202) comprising:
a sensing portion comprising a substantially V-shaped portion (204), the substantially V-shaped portion having an apex (209) that extends along the entire ring width, the apex (209) comprising an angle of between 85° and 120°;
one or more sensing means (206) disposed in the V-shaped portion (204); and
a pad (208) disposed in the aperture and opposing the sensing portion,
wherein the pad (208) is configured to urge the finger against the one or more sensing means (206) in the V-shaped portion (204) when the finger is received in the aperture, such that a central position (153) on an underside of the finger (160) may be substantially aligned with the apex (209) of the substantially V-shaped portion (204).

2. The ring (200) of claim 1, wherein the pad (208) is resiliently deformable.

3. The ring of claim 1 or 2, wherein the pad (208) is removable.

4. The ring (200) of any of claims 1 to 3, wherein the pad (208) is receivable in the ring (200) by means of cooperating male pips (506, 508) and female detents (510, 512) in the pad (208) and the ring (200).

5. The ring (200) of any preceding claim, wherein the pad (208) comprises a silicone elastomer.

6. The ring (200) of any preceding claim, wherein the pad (208) comprises a plurality of projections (210) for contacting the finger.

7. The ring (200) of any preceding claim, comprising an active input means for engaging by the user.

8. The ring (200) of any preceding claim, wherein the one or more sensing means (206) comprises any one or more: a temperature sensor (206E), a galvanic skin response "GSR" sensor (205A, 205B), an optical sensor (205C, 206d), a light sensor, an accelerometer, a gyroscope and a force sensor.

9. The ring (200) of any preceding claim, comprising one or more sensors for measuring one or more attributes of an environment proximal to the ring (200).

10. The ring (200) of any preceding claim, comprising a memory means for storing sensed information.

11. The ring (200) of any preceding claim, comprising means for transmitting sensed information to a remote receiving device.

12. A kit comprising:
the ring (200) of any of the preceding claims ; and
one or more replacement pads (208).

13. The kit of claim 12, wherein the one or more replacement pads (208) comprise different dimensions relative to each other.

## Patentansprüche

1. Ring (200) zum Erfassen von einem oder mehreren physiologischen und/oder Verhaltensparametern eines Benutzers, wobei der Ring (200) Folgendes umfasst:
eine Öffnung (202) zum Aufnehmen eines Fingers (100) des Benutzers dadurch, wobei die Öffnung (202) Folgendes umfasst:
einen Erfassungsabschnitt, der einen im Wesentlichen V-förmigen Abschnitt (204) umfasst, wobei der im Wesentlichen V-förmige Abschnitt einen Scheitelpunkt (209) aufweist, der sich entlang der gesamten Ringbreite erstreckt, wobei der Scheitelpunkt (209) einen Winkel zwischen 85° und 120° umfasst;
ein oder mehrere Erfassungsmittel (206), die in dem V-förmigen Abschnitt (204) angeordnet sind; und
ein Kissen (208), das in der Öffnung angeordnet ist und dem Erfassungsabschnitt gegenüberliegt, wobei das Kissen (208) dazu konfiguriert ist, den Finger gegen das eine oder die mehreren Erfassungsmittel (206) in dem V-förmigen Abschnitt (204) zu drücken, wenn der Finger in der Öffnung aufgenommen ist, sodass eine zentrale Position (153) an einer Unterseite des Fingers (160) im Wesentlichen mit dem Scheitelpunkt (209) des im Wesentlichen V-förmigen Abschnitts (204) ausgerichtet sein kann.

2. Ring (200) nach Anspruch 1, wobei das Kissen (208) elastisch verformbar ist.

3. Ring nach Anspruch 1 oder 2, wobei das Kissen (208) entfernbar ist.

4. Ring (200) nach einem der Ansprüche 1 bis 3, wobei das Kissen (208) mittels zusammenwirkender männlicher Kerben (506, 508) und weiblicher Arretierungen (510, 512) in dem Kissen (208) und dem Ring (200) in dem Ring (200) aufnehmbar ist.

5. Ring (200) nach einem der vorhergehenden Ansprüche, wobei das Kissen (208) ein Silikonelastomer umfasst.

6. Ring (200) nach einem der vorhergehenden Ansprüche, wobei das Kissen (208) eine Vielzahl von Vorsprüngen (210) zum Inkontaktbringen mit dem Finger umfasst.

7. Ring (200) nach einem vorhergehenden Anspruch, umfassend ein aktives Eingabemittel zum Ineingriffnehmen durch den Benutzer.

8. Ring (200) nach einem der vorhergehenden Ansprüche, wobei das eine oder die mehreren Erfassungsmittel (206) ein beliebiges oder mehrere beliebige von Folgenden umfassen: einem Temperatursensor (206E), einem galvanischen Hautreaktionssensor, "GSR"-Sensor, (205A, 205B), einem optischen Sensor (205C, 206d), einem Lichtsensor, einem Beschleunigungsmesser, einem Gyroskop und einem Kraftsensor.

9. Ring (200) nach einem vorhergehenden Anspruch, umfassend einen oder mehrere Sensoren zum Messen eines oder mehrerer Attribute einer Umgebung in der Nähe des Rings (200).

10. Ring (200) nach einem vorhergehenden Anspruch, umfassend ein Speichermittel zum Speichern von erfassten Informationen.

11. Ring (200) nach einem vorhergehenden Anspruch, umfassend ein Mittel zum Übertragen von erfassten Informationen an eine entfernte Empfangsvorrichtung.

12. Kit, umfassend:
den Ring (200) nach einem der vorhergehenden Ansprüche; und
ein oder mehrere Ersatzkissen (208).

13. Kit nach Anspruch 12, wobei das eine oder die mehreren Ersatzkissen (208) unterschiedliche Abmessungen bezogen aufeinander umfassen.

## Revendications

1. Bague (200) pour détecter un ou plusieurs paramètres physiologiques et/ou de comportement d'un utilisateur, la bague (200) comprenant :
une ouverture (202) pour recevoir un doigt (100) de l'utilisateur à travers celle-ci, l'ouverture (202) comprenant :
une partie de détection comprenant une partie sensiblement en forme de V (204), la partie sensiblement en forme de V ayant un sommet (209) qui s'étend sur toute la largeur de la bague, le sommet (209) comprenant un angle situé entre 85° et 120° ;
un ou plusieurs moyens de détection (206) disposés dans la partie en forme de V (204) ; et
un tampon (208) disposé dans l'ouverture et faisant face à la partie de détection,
dans laquelle le tampon (208) est conçu pour pousser le doigt contre l'un ou plusieurs moyens de détection (206) dans la partie en forme de V (204) lorsque le doigt est reçu dans l'ouverture, de sorte qu'une position centrale (153) sur un côté inférieur du doigt (160) puisse être sensiblement alignée au sommet (209) de la partie sensiblement en forme de V (204).

2. Bague (200) selon la revendication 1, dans laquelle le tampon (208) est élastiquement déformable.

3. Bague selon la revendication 1 ou 2, dans laquelle le tampon (208) est amovible.

4. Bague (200) selon l'une quelconque des revendications 1 à 3, dans laquelle le tampon (208) peut être reçu dans la bague (200) au moyen d'ergots mâles coopérants (506, 508) et de crans femelles (510, 512) dans le tampon (208) et la bague (200).

5. Bague (200) selon une quelconque revendication précédente, dans laquelle le tampon (208) comprend un élastomère de silicone.

6. Bague (200) selon une quelconque revendication précédente, dans lequel le tampon (208) comprend une pluralité de saillies (210) pour entrer en contact avec le doigt.

7. Bague (200) selon une quelconque revendication précédente, comprenant un moyen d'entrée actif destiné à être engagé par l'utilisateur.

8. Bague (200) selon une quelconque revendication précédente, dans laquelle l'un ou plusieurs moyens de détection (206) comprennent un ou plusieurs parmi : un capteur de température (206E), un capteur de réponse galvanique de la peau « GSR » (205A, 205B), un capteur optique (205C, 206d), un capteur de lumière, un accéléromètre, un gyroscope et un capteur de force.

9. Bague (200) selon une quelconque revendication précédente, comprenant un ou plusieurs capteurs pour mesurer un ou plusieurs attributs d'un environnement à proximité de la bague (200).

10. Bague (200) selon une quelconque revendication précédente, comprenant un moyen de mémoire pour stocker des informations détectées.

11. Bague (200) selon une quelconque revendication précédente, comprenant un moyen pour transmettre les informations détectées à un dispositif de réception distant.

12. Kit comprenant :
la bague (200) selon l'une quelconque des revendications précédentes ; et
un ou plusieurs tampons de remplacement (208).

13. Kit selon la revendication 12, dans lequel l'un ou plusieurs tampons de remplacement (208) comprennent des dimensions différentes les uns des autres.
